# EUROPÄISCHE PATENTSCHRIFT

(11) **EP 1 453 814 B1**
(45) Veröffentlichungstag und Bekanntmachung des Hinweises auf die Patenterteilung: **28.09.2005**
(21) Anmeldenummer: 02792954.6
(22) Anmeldetag: 10.12.2002
(51) Int. Cl.: C07D 251/62

(54) **VERFAHREN ZUR REINIGUNG EINER MELAMINSCHMELZE**
METHOD FOR PURIFYING A MELAMINE MELT
PROCEDE DE PURIFICATION DE MELANINE FONDUE

(30) Priorität: 12.12.2001 AT 19492001
(43) Veröffentlichungstag der Anmeldung: 08.09.2004
(73) Patentinhaber: AMI Agrolinz Melamine International GmbH, 4021 Linz (AT)
(72) Erfinder: COUFAL, Gerhard, A-4060 Leonding (AT)
(74) Vertreter: Gross, Felix
(86) Internationale Anmeldenummer: PCT/EP2002/014007
(87) Internationale Veröffentlichungsnummer: WO 2003/053943

(56) Entgegenhaltungen:
- US-A- 3 116 294
- US-A- 3 207 744

## Beschreibung

Die Erfindung betrifft die Herstellung von reinem Melamin durch Pyrolyse von Harnstoff in einem Hochdruckprozess und Reinigen der gebildeten Melaminschmelze durch geteiltes Strippen.

Bei den Hochdruckverfahren zur Herstellung von Melamin wird Harnstoff in einer endothermen Flüssigphasenreaktion zu Melamin umgesetzt. Dabei entstehen pro Mol Melamin 3 Mol CO₂ und 6 Mol NH₃, wobei das flüssige Melamin je nach Druck- und Temperaturbedingungen im Reaktor zusätzliche unterschiedliche Mengen gelöstes NH₃ und CO₂ sowie höhermolekulare und niedermolekulare Nebenprodukte und nicht umgesetzten Harnstoff enthält.
Aus US-A 3,116,294 ist bekannt, dass durch Strippen einer Roh-Melaminschmelze mit gasförmigem NH₃ im Gegenstrom das CO₂ aus dem Roh-Melamin entfernt werden kann.
In WO 00/21940 ist beschrieben, dass das Strippen vorteilhaft in einer mit flüssigem Melamin gefüllten, und nicht in einer gasgefüllten Säule erfolgt. Weiters ist es möglich, die Schmelze in der Blasensäule auf eine Temperatur zu kühlen, die 5 - 20 °C über dem Schmelzpunkt des Melamins liegt.

Unerwarteterweise wurde nun ein Verfahren gefunden, das es ermöglicht, durch geteilte NH₃-Zugabe in den Stripper eine höhere Reinheit des Melamins zu erhalten.

Gegenstand der Erfindung ist demnach ein Verfahren zur Herstellung von reinem Melamin in einem Hochdruckverfahren durch Pyrolyse von Harnstoff, das dadurch gekennzeichnet ist, dass die gebildete Melaminschmelze einer Strippeinheit zugeführt wird, in der die Melaminschmelze im Gegenstrom
- in einer ersten Stufe mit heißem frischem gasförmigem NH₃ und zusätzlich mit NH₃ aus der zweiten Stufe in Kontakt gebracht wird, wobei die Temperatur der Schmelze gleich bleibt oder höher wird
- in einer zweiten Stufe mit kaltem gasförmigem NH₃ derart in Kontakt gebracht wird, dass sie dabei auf eine Temperatur, die 1 - 30 ° über dem druckabhängigen Schmelzpunkt des Melamins liegt, gekühlt wird und gegebenenfalls
- in einer dritten Stufe 10 min - 10 Stunden verweilen gelassen wird,
- worauf die Melaminschmelze in beliebiger Weise aufgearbeitet wird.

Zur Durchführung des erfindungsgemäßen Verfahrens wird Harnstoff bei einer Temperatur von 325 - 450 °C, bevorzugt von 350 - 425 °C und einem Druck von 50 - 450 bar, bevorzugt von 50 - 250 bar zu flüssigem Melamin und Offgas umgesetzt. Dabei wird dem Melaminreaktor zur Vermeidung der Bildung von Nebenprodukten oder je nach Bauweise des Reaktors auch zur besseren Durchmischung im Reaktor überschüssiges NH₃-Gas von bis zu 10 Mol NH₃, bevorzugt von bis zu 2 Mol NH₃ pro Mol Harnstoff zugesetzt.
Nach dem erfindungsgemäßen Verfahren ist es nicht notwendig, das im Reaktor gebildete Reaktionsgemisch einem Separator zuzuführen und dort die flüssige Phase, die Melaminschmelze, von der gasförmigen Phase, den Offgasen, vollständig zu trennen.
Es genügt, die Offgase am Reaktorkopf abzuziehen, und die flüssige Phase, die noch Anteile von gelösten Offgasen enthält, direkt der Strippeinheit zuzuführen.

Die am Reaktorkopf abgezogenen Offgase, welche aus gasförmigem NH₃, CO₂ und geringen Teilen an gasförmigen Melamin bestehen, werden gegebenenfalls mit den Offgasen aus weiteren Hochdruckteilen der Melaminanlage, welche ebenfalls aus gasförmigem NH₃, CO₂ und geringen Teilen aus gasförmigem Melamin bestehen, einem Harnstoffwäscher zugeführt. Im Harnstoffwäscher befindet sich Harnstoffschmelze, die das im heißen Offgas enthaltene gasförmige Melamin auswäscht, und sich dabei erwärmt, während das Offgas von Melamin gereinigt und abgekühlt wird. Die vorgewärmte, melaminhältige Harnstoffschmelze wird dann dem Melaminreaktor zugeführt und zu Melamin umgesetzt.

Die den Reaktor verlassende Melaminschmelze enthält zusätzlich unterschiedliche Mengen gelöstes NH₃ und CO₂ sowie höhermolekulare und niedermolekulare Nebenprodukte und nicht umgesetzten Harnstoff. Das CO₂ sowie die Nebenprodukte und der nicht umgesetzte Harnstoff sollen möglichst vollständig aus der Melaminschmelze entfernt werden.
Dies erfolgt erfindungsgemäß durch geteiltes Strippen in einer Strippeinheit, in der die Melaminschmelze im Gegenstrom in der ersten Stufe mit heißem frischen NH₃ und zusätzlich mit heißem NH₃ aus der zweiten Stufe gestrippt wird, wobei die Temperatur der Schmelze gleich bleibt oder auch höher wird und die so vorgereinigte Melaminschmelze in der zweiten Stufe mit kaltem gasförmigem NH₃ derart gestrippt wird, dass die Schmelze auf eine Temperatur, die möglichst knapp über dem druckabhängigen Schmelzpunkt des Melamins liegt, gekühlt wird. Je nach Bauart der Anlage ist diese Temperatur 1 - 30 °C, bevorzugt 1 - 20 °, besonders bevorzugt 1 - 10 °C über dem druckabhängigen Schmelzpunkt des Melamins.

Die Melaminschmelze steht unter einem NH₃-Druck von 50 - 450 bar, bevorzugt ist es der Reaktordruck. Es ist jedoch auch möglich, das Strippen bei einem Druck deutlich unterhalb des Reaktordruckes erfolgreich durchzuführen. Die Temperatur der in die Strippeinheit eintretenden Melaminschmelze ist in einer bevorzugten Ausführungsform etwa gleich der Reaktortemperatur. Es ist jedoch auch möglich, die Temperatur zu erhöhen oder zu erniedrigen.
Zum Strippen in der ersten Stufe wird frisches Ammoniak mit einer Temperatur zugeführt, die etwa gleich der Temperatur der in der ersten Stufe vorhandenen Schmelze ist. Es ist jedoch auch möglich, NH₃ einer etwas höheren Temperatur zuzuführen, sodass insgesamt die Schmelzetemperatur in der ersten Stufe erhöht wird.
Gleichzeitig mit dem frischen heißen NH₃ wird auch das nach dem Durchleiten durch die zweite Stufe erhaltene, nunmehr erwärmte NH₃ in die 1. Stufe eingeführt, sodass insgesamt für den Strippvorgang in der 1. Stufe die gesamte in der Strippeinheit eingesetzte NH₃-Menge in der zum optimalen Strippen nötigen hohen Temperatur zur Verfügung steht.
Die Eintrittsstelle des frischen heißen NH₃ liegt in jener Zone der Strippeinheit, in der die in der zweiten Stufe gekühlte Melaminschmelze etwa die Eingangstemperatur der Melaminschmelze in die erste Stufe erreicht hat.

Je nach den eingesetzten Mengen, Temperaturen und konstruktiven Gegebenheiten der Strippeinheit ergibt sich die Lage jener Zone, in der praktisch keine Kühlung der Melaminschmelze mehr stattfindet, und in der die erste Stufe beginnt und das frische heiße NH₃ zugeführt wird.
Die Zufuhr des frischen heißen NH₃-Gases kann an einer oder mehreren Stellen erfolgen.

Die Einleitung des kalten NH₃ in der zweiten Stufe erfolgt am Boden der Strippeinheit in einer Menge und einer Temperatur, die die Melaminschmelze auf eine Temperatur, die möglichst knapp über ihrem jeweiligen Schmelzpunkt liegt, kühlt. Es muss jedoch darauf Bedacht genommen werden, dass dabei die Temperatur bei etwaigen Schwankungen im Betrieb nicht unter den Schmelzpunkt sinkt, und Melamin kristallisiert.
Die Temperatur des kalten NH₃ liegt im Bereich von 150 - 300 °C, bevorzugt von 150 - 200 °C, die Menge des nötigen kalten NH₃ ist abhängig von der Eingangstemperatur der Melaminschmelze, von der durchgesetzten Menge, von der Größe und Bauart der Strippeinheit, und kann demgemäß in einem großen Bereich schwanken.
Beim Durchgang durch die zweite Stufe nimmt das kalte NH₃ die beim Abkühlen der Melaminschmelze abgeführte Wärmemenge auf und wird dabei selbst erwärmt.
In dieser zweiten Stufe werden vor allem höhermolekulare Nebenprodukte entfernt.

In einer weiteren Ausführungsform der Erfindung befindet sich unterhalb der zweiten Stufe Raum für eine dritte Stufe, in der die auf eine Temperatur, die möglichst knapp oberhalb des Melaminschmelzpunktes liegt, gebrachte Melaminschmelze unter dem vorhandenen NH₃-Druck verweilen gelassen wird. Dadurch kann ein zusätzlicher Aging-Effekt erreicht werden.

Die am Ausgang der zweiten oder dritten Stufe erhaltene reine Melaminschmelze kann in beliebiger Weise weiter aufgearbeitet und verfestigt werden, beispielsweise durch Entspannen der Schmelze, durch Verfestigen in einer Wirbelschicht, durch Quenchen mit Wasser, mit flüssigem oder gasförmigem Ammoniak, oder durch Sublimieren und anschließendes Desublimieren aus der Gasphase.

Das erfindungsgemäße Verfahren eignet sich für alle Arten von Gas-Flüssigkeitskolonnen. Es können die unterschiedlichsten Arten von Füllungen wie Lochbleche, Siebböden, Ventilböden oder Packungen wie z.B. Sulzer Packungen eingesetzt werden.

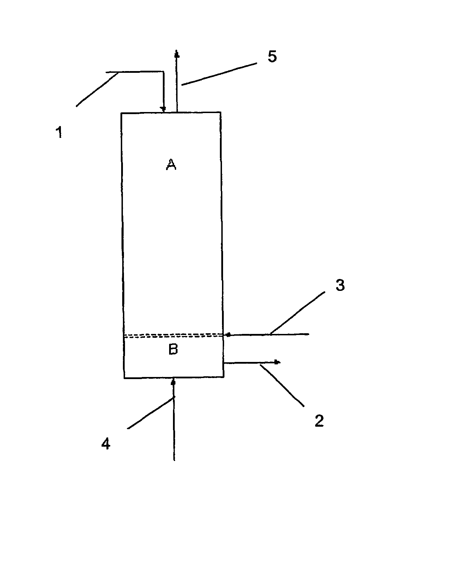

Abbildung 1 zeigt die schematische Darstellung einer Strippeinheit, in der A die erste Stufe der Strippeinheit, B die zweite Stufe der Strippeinheit, 1 die in die erste Stufe eintretende Melaminschmelze, 2 die aus der zweiten Stufe austretende Melaminschmelze, 3 heißes gasförmiges NH₃, 4 kaltes NH₃ und 5 austretendes NH₃ bedeuten.

Das nachfolgende Beispiel 1 zeigt, dass mit Hilfe der erfindungsgemäßen geteilten Zugabe von NH₃ unterschiedlicher Temperatur bei gleicher NH₃-Strippmenge Melamin höherer Reinheit erhalten wird, als bei ungeteilter NH₃-Zugabe bei konstanter Temperatur am Boden des Reaktors nach Vergleichsbeispiel 1. Vergleichsbeispiel 2 zeigt, dass, falls die Säule ein Temperaturprofil zwischen Eingang und Ausgang der Melaminschmelze aufweist, es nicht möglich ist, die zur Herstellung von reinem Melamin nötige Menge an "kaltem" NH₃ einzubringen, da dann die Temperatur des Melamins am Ausgang der Strippeinheit nicht mehr auf der erforderlichen Temperatur gehalten werden kann. Es wird Melamin noch geringerer Qualität erhalten.

### Beispiel 1: Geteilte NH₃-Zugabe

In eine mit Sulzer Packungen gefüllte Säule von 1 Meter Länge und 8 cm Durchmesser, die unter einem NH₃-Druck von 180 bar steht, wurden am Kopf der Säule 4 kg Melamin/Stunde mit einer Temperatur von 370 °C eingebracht. Das Melamin hatte einen Gehalt an sauerstoffhältigen Komponenten (CO₂, Ammelin, Ammelid, Ureidomelamin und Isocyansäure) von 2,9 Gew.%. Am Boden der Säule wurden 0,43 kg NH₃ einer Temperatur von 160 °C, und am Beginn des Temperaturbereichs der Melaminschmelze von 370 °C wurden 2,9 kg/h NH₃ einer Temperatur von 370 °C eingebracht.
Am Boden der Säule, wo eine Temperatur von 340 °C vorliegt, wurde Melamin mit einer Reinheit von 99,6 Gew.% und einem Gehalt an sauerstoffhältigen Komponenten von 0,21 Gew.% erhalten.

### Vergleichsbeispiel 1: Ungeteilte NH₃-Zugabe

Wie in Beispiel 1 wurden am Kopf der Säule 4 kg Melamin eines Gehaltes an sauerstoffhältigen Komponenten von 2,9 Gew.% und einer Temperatur von 370 °C eingetragen. Es wurde jedoch die Gesamtmenge an NH₃ von 3,33 kg/h am Boden des Reaktors und mit einer Temperatur von 370 °C eingebracht.
Die Reinheit der am Boden des Reaktors erhaltenen Melaminschmelze betrug 99,0 Gew.%, ihr Gehalt an sauerstoffhältigen Komponenten 0,22 Gew.%.

### Vergleichsbeispiel 2: Ungeteilte NH₃-Zugabe

Wie in Beispiel 1 wurde am Kopf der Säule 4 kg Melamin/h eines Gehaltes an sauerstoffhältigen Komponenten von 2,9 Gew.% und einer Temperatur von 370 °C eingetragen. Am Boden der Strippkolonne wurde solange NH₃ einer Temperatur von 330 °C eingetragen, bis am Boden der Säule eine Temperatur von 340 °C vorlag.

Der Gehalt der am Boden des Reaktors erhaltenen Melaminschmelze an sauerstoffhältigen Komponenten war 0,45 Gew.%, die Reinheit des Melamins betrug 99,2 Gew.%.

## Patentansprüche

1. Verfahren zur Herstellung von reinem Melamin in einem Hochdruckverfahren durch Pyrolyse von Harnstoff, **dadurch gekennzeichnet, dass** die gebildete Melaminschmelze einer Strippeinheit zugeführt wird, in der die Melaminschmelze im Gegenstrom
- in einer ersten Stufe mit heißem frischem gasförmigem NH₃ und zusätzlich mit NH₃ aus der zweiten Stufe in Kontakt gebracht wird, wobei die Temperatur der Schmelze gleich bleibt oder höher wird
- in einer zweiten Stufe mit kaltem gasförmigem NH₃ derart in Kontakt gebracht wird, dass sie dabei auf eine Temperatur, die 1 -30 ° über dem druckabhängigen Schmelzpunkt des Melamins liegt, gekühlt wird
- worauf die Melaminschmelze in beliebiger Weise aufgearbeitet wird.

2. Verfahren nach Anspruch 1, **dadurch gekennzeichnet, dass** die Melaminschmelze nach der zweiten Stufe in einer dritten Stufe 10 min -10 Stunden verweilen gelassen wird

3. Verfahren nach Anspruch 1 oder 2, **dadurch gekennzeichnet, dass** die Melaminschmelze in der zweiten Stufe mit kaltem gasförmigem NH₃ derart in Kontakt gebracht wird, dass sie auf eine Temperatur, die 1 - 20 °C über dem druckabhängigen Schmelzpunkt des Melamins liegt, gebracht wird.

4. Verfahren nach Anspruch 1, **dadurch gekennzeichnet, dass** die Melaminschmelze in der zweiten Stufe mit kaltem gasförmigem NH₃ derart in Kontakt gebracht wird, dass sie auf eine Temperatur, die 1 -10 °C über dem druckabhängigen Schmelzpunkt des Melamins liegt, gebracht wird.

5. Verfahren nach Anspruch 1, **dadurch gekennzeichnet, dass** die Melaminschmelze direkt aus dem Reaktor kommt.

6. Verfahren nach Anspruch 1, **dadurch gekennzeichnet, dass** die Temperatur der Melaminschmelze in der ersten Stufe gleich der Reaktortemperatur ist.

7. Verfahren nach Anspruch 1, **dadurch gekennzeichnet, dass** die Zufuhr von heißem frischem gasförmigem NH₃ in der ersten Stufe an mehreren Stellen erfolgt.

8. Verfahren nach Anspruch 1, **dadurch gekennzeichnet, dass** die Temperatur des in der zweiten Stufe zugeführten kalten gasförmigen NH₃ 150 - 300 °C beträgt.

9. Verfahren nach Anspruch 1, **dadurch gekennzeichnet, dass** die Temperatur des in der zweiten Stufe zugeführten kalten gasförmigen NH₃ 150 - 200 ° beträgt.

## Claims

1. Process for preparing pure melamine in a highpressure process by pyrolysis of urea, **characterized in that** the melamine melt formed is fed into a stripping unit in which the melamine melt is, in countercurrent,
- in a first stage brought into contact with hot fresh gaseous NH₃ and additionally with NH₃ from the second stage, with the temperature of the melt remaining the same or becoming higher,
- in a second stage brought into contact with cold gaseous NH₃ in such a way that it is cooled to a temperature which is 1-30°C above the pressure-dependent melting point of melamine,
- whereupon the melamine melt is worked up in any desired way.

2. Process according to Claim 1, **characterized in that** the melamine melt after the second stage is left to rest for 10 minutes - 10 hours in a third stage.

3. Process according to Claim 1 or 2, **characterized in that** the melamine melt is in the second stage brought into contact with cold gaseous NH₃ in such a way that it is brought to a temperature which is 1-20°C above the pressure-dependent melting point of melamine.

4. Process according to Claim 1, **characterized in that** the melamine melt is in the second stage brought into contact with cold gaseous NH₃ in such a way that it is brought to a temperature which is 1-10°C above the pressure-dependent melting point of melamine.

5. Process according to Claim 1, **characterized in that** the melamine melt comes directly from the reactor.

6. Process according to Claim 1, **characterized in that** the temperature of the melamine melt in the first stage is the same as the reactor temperature.

7. Process according to Claim 1, **characterized in that** the introduction of hot fresh gaseous NH₃ into the first stage is effected at a plurality of points.

8. Process according to Claim 1, **characterized in that** the temperature of the cold gaseous NH₃ introduced in the second stage is 150-300°C.

9. Process according to Claim 1, **characterized in that** the temperature of the cold gaseous NH₃ introduced in the second stage is 150-200°C.

## Revendications

1. Procédé en vue de la fabrication de mélamine pure dans un procédé haute pression par pyrolyse d'urée, **caractérisé en ce que** la masse en fusion de mélamine formée est alimentée à une installation de stripage dans laquelle la masse en fusion de mélanine à contre-courant
- est mise en contact, dans une première étape, avec du NH₃ gazeux frais chaud et, en plus, avec du NH₃ en provenance de la deuxième étape, la température de la masse en fusion restant égale ou supérieure,
- est amenée en contact, dans une deuxième étape, avec du NH₃ gazeux froid, de telle sorte qu'elle se refroidisse, en l'occurrence, à une température, qui se situe de 1 - 30 °C au-dessus du point de fusion de la mélamine, dépendant de la pression,
- à la suite de quoi, la masse en fusion de mélamine est traitée d'une manière quelconque.

2. Procédé selon la revendication 1, **caractérisé en ce que** la masse en fusion de mélamine, dans une troisième étape, est laissée au repos, après la deuxième étape, pendant une durée de 10 minutes à 10 heures.

3. Procédé selon la revendication 1 ou 2, **caractérisé en ce que** la masse en fusion de mélamine est mise en contact, dans la deuxième étape, avec du NH₃ gazeux froid, de telle sorte qu'elle est amenée à une température qui se situe de 1 - 20 °C au-dessus du point de fusion de la mélamine, dépendant de la pression.

4. Procédé selon la revendication 1, **caractérisé en ce que** la masse en fusion de mélamine est mise en contact, dans la deuxième étape, avec du NH₃ gazeux froid, de telle sorte qu'elle est amenée à une température qui se situe de 1 - 10 °C au-dessus du point de fusion de la mélamine, dépendant de la pression.

5. Procédé selon la revendication 1, **caractérisé en ce que** la masse en fusion de mélamine provient directement du réacteur.

6. Procédé selon la revendication 1, **caractérisé en ce que** la température de la masse en fusion de mélamine dans la première étape est égale à la température du réacteur.

7. Procédé selon la revendication 1, **caractérisé en ce que** l'amenée de NH₃ gazeux frais chaud se fait dans la première étape à plusieurs endroits.

8. Procédé selon la revendication 1, **caractérisé en ce que** la température du NH₃ gazeux froid alimenté dans la deuxième étape est de 150 - 300 °C.

9. Procédé selon la revendication 1, **caractérisé en ce que** la température du NH₃ gazeux froid alimenté dans la deuxième étape est de 150 - 200 °C.
